# EUROPÄISCHE PATENTSCHRIFT

(11) **EP 1 845 881 B1**
(45) Veröffentlichungstag und Bekanntmachung des Hinweises auf die Patenterteilung: **28.07.2010**
(21) Anmeldenummer: 05824373.4
(22) Anmeldetag: 24.12.2005
(51) Int. Cl.: A61B 19/00, A61F 2/46

(54) **Vorrichtung ZUR ANZEIGE DER POSITION UND ORIENTIERUNG EINES CHIRURGISCHEN raspelähnlichen WERKZEUGES oder eines Femurimplantats**
Device FOR DISPLAY OF THE POSITION AND ORIENTATION OF A rasplike SURGICAL TOOL or of a femoral implant
Dispositif POUR AFFICHER LA POSITION ET L'ORIENTATION d'un implant fémoral ou D'UN OUTIL CHIRURGICAL du type râpe

(30) Priorität: 17.01.2005 DE 102005003318
(43) Veröffentlichungstag der Anmeldung: 24.10.2007
(73) Patentinhaber: Aesculap AG, 78532 Tuttlingen (DE)
(72) Erfinder: MOLLARD, Benoît, 38130 Echirolles (FR); PEROT, Cyril, F-38700 La Tronche (FR); KAMMERZELL, Sergej, 78234 Engen (DE)
(74) Vertreter: Hoeger, Stellrecht & Partner Patentanwälte
(86) Internationale Anmeldenummer: PCT/EP2005/014039
(87) Internationale Veröffentlichungsnummer: WO 2006/074807

(56) Entgegenhaltungen:
- WO-A-2004/030556
- DE-A1- 10 306 793
- US-A1- 2003 153 829
- US-A1- 2004 077 940
- US-A1- 2004 147 926

## Beschreibung

Die Erfindung betrifft eine Vorrichtung zur Anzeige der Position und Orientierung eines raspelähnlichen Werkzeuges zur Vorbereitung eines Femurs für ein mit einer Implantat-Gelenkkugel in das Acetabulum der Pelvis oder in eine in die Pelvis eingesetzte Gelenkpfanne eingreifendes Femurimplantat, umfassend ein derartiges Werkzeug, wobei das raspelähnliche Werkzeug dem Schaft des Femurimplantates sehr ähnlich ausgebildet ist und geeignet ist, die Markhöhle formgerecht so vorzubereiten, dass beim Einführen des Schaftes des Femurimplantates die Positionierung des Femurimplantates im Femur durch die Geometrie des vorbereiteten Markraumhohlraumes bestimmt wird, ein Navigationssystem mit dem Femur, der Pelvis sowie dem Werkzeug oder dem Femurimplantat zugeordneten Markierelementen zur Lagebestimmung dieser Teile, und eine dem Navigationssystem zugeordnete Datenverarbeitungsanlage, die geeignet ist, eine Anzeige zu aktivieren.

Außerdem betrifft die Erfindung eine Vorrichtung zur Anzeige der Position und Orientierung eines in einen Femur einzusetzenden Femurimplantates relativ zum Femur und zum Mittelpunkt der Femur-Gelenkkugel, umfassend ein derartiges Femurimplantat, ein Navigationssystem mit dem Femur, der Pelvis sowie dem Werkzeug oder und dem Femurimplantat zugeordneten Markiertementen zur Lagebestimmung dieser Teile, und mit einer dem Navigationssystem zugeordneten Datenverarbeitungsanlage, die eine Anzeige aktiviert.

Bei Hüftgelenkoperationen ist es notwendig, ein Femurimplantat in den Femur einzusetzen, nachdem der Kopf des Femurs entfernt worden ist. Das Femurimplantat ist dem natürlichen Knochenverlauf in der Regel angepasst und weist einen in den Femur einführbaren Schaft, einen schräg davon abgehenden Schenkelhals und daran eine Implantat-Gelenkkugel auf, die in die Gelenkpfanne der Pelvis eingreift, entweder in die natürliche oder in den meisten Fällen in eine künstliche Gelenkpfanne.

Zum Einsetzen des Femurimplantates in den Femur muss dieser vorbereitet werden, und es ist üblich, dies mit Hilfe raspelähnlicher Werkzeuge zu bewerkstelligen, die den Markraum des Femurs so vorbereiten, dass der Schaft des Femurimplantates passrichtig in den Femur eingesetzt werden kann, d.h. so, dass das Implantat relativ zum Femur die gewünschte Position und Orientierung einnimmt, so dass beim Eingreifen der Implantat-Gelenkkugel des Femurimplantates in das Acetabulum eine korrekte Positionierung des Femurs erfolgt.

Der Operateur muss dabei das raspelähnliche Werkzeug oder beim Einsetzen des Femurimplantates dieses selbst relativ zum Femur korrekt führen bzw. platzieren, und dazu ist es bekannt, dem Operateur auf einem Bildschirm anzuzeigen, welche Winkel das raspelähnliche Werkzeug oder das Femurimplantat relativ zum Femur einnehmen. Damit ist die Operation zwar durchführbar, die Anzeige von numerischen Winkelangaben ist aber nicht ohne weiteres verständlich und muss vom Operateur erst in die notwendigen Manipulationen umgesetzt werden.

Es ist Aufgabe der Erfindung, eine Vorrichtung zur Anzeige der eingangs beschriebenen Art so auszubilden, dass dem Operateur die Veränderung von Position und Orientierung, also der Lage des den Femur vorbereitenden raspelähnlichen Werkzeuges bzw. des Femurimplantates so deutlich dargestellt wird, dass er ohne weitere Überlegung durch Änderung der Lage die gewünschte Position erreichen und an einer Anzeige auch ablesen kann.

Diese Aufgabe wird bei einer Vorrichtung zur Anzeige der Position und Orientierung eines raspelähnliches Werkzeuges der eingangs beschriebenen Art erfindungsgemäß dadurch gelöst, dass die Datenverarbeitungsanlage so programmiert ist, dass sie zum Auffinden einer Transversalebene des Femurs aus der Position und Orientierung des Mittelpunktes der Gelenkkugel des natürlichen Femurkopfes und des Kniegelenks als Verbindungslinie dieser beiden Punkte eine mechanische Femurachse bestimmt und die Transversalebene des Femurs als senkrecht auf der mechanischen Femurachse und durch den Mittelpunkt der Gelenkkugel des natürlichen Femurkopfes gehend bestimmt, und dass sie auf der Anzeige in dieser Transversalebene des Femurs in Höhe des Mittelpunktes der Gelenkkugel des natürlichen Femurkopfes diesen Mittelpunkt und eine Schnittlinie der Transversalebene des Femurs und einer Instrumentenebene darstellt, wobei die Datenverarbeitungsanlage so programmiert ist, dass sie die Instrumentenebene so auswählt, dass die Projektion der Längsachse eines den Schaft des raspelähnlichen Werkzeuges mit der Implantat-Gelenkkugel verbindenden Haltearmes auf die Transversalebene des Femurs in der Instrumentenebene liegt und dass die Instrumentenebene durch den Mittelpunkt der Implantat-Gelenkkugel hindurchgeht.

Bei einer Vorrichtung zur Anzeige der Position und Orientierung eines Femurimplantates der eingangs beschriebenen Art wird diese Aufgabe erfindungsgemäß dadurch gelöst, dass die Datenverarbeitungsanlage so programmiert ist, dass sie zum Auffinden einer Transversalebene des Femurs aus der Position und Orientierung des Mittelpunktes der Gelenkkugel des natürlichen Femurkopfes und des Kniegelenks als Verbindungslinie dieser beiden Punkte eine mechanische Femurachse bestimmt und die Transversalebene des Femurs als senkrecht auf der mechanischen Femurachse und durch den Mittelpunkt der Gelenkkugel des natürlichen Femurkopfes gehend bestimmt, und dass sie auf der Anzeige in dieser Transversalebene des Femurs in Höhe des Mittelpunktes der Gelenkkugel des natürlichen Femurkopfes diesen Mittelpunkt und eine Schnittlinie der Transversalebene des Femurs und einer Instrumentenebene darstellt, wobei die Datenverarbeitungsanlage so programmiert ist, dass sie die Instrumentenebene so auswählt, dass die Projektion der Längsachse eines den Schaft eines des Femurimplantates mit der Implantat-Gelenkkugel verbindenden Haltearmes auf die Transversalebene des Femurs in der Instrumentenebene liegt und dass die Instrumentenebene durch den Mittelpunkt der Implantat-Gelenkkugel hindurchgeht.

Der Operateur kann somit auf dem Bildschirm einmal den Mittelpunkt der Gelenkkugel des natürlichen Femurkopfes sehen und zum anderen die Schnittlinie, die ihm anzeigt, unter welchem Winkel in der Transversalebene die Instrumentenebene schneidet. Die Instrumentenebene kann dabei eine Ebene eines chirurgischen, raspelähnlichen Instrumentes sein, die Instrumentenebene kann aber auch eine Ebene des Femurimplantates sein.

Insbesondere kann vorgesehen sein, dass man die Instrumentenebene so wählt, dass sie die Mittelebene des raspelähnlichen Werkzeuges ist. Häufig haben die Schäfte und dann auch die entsprechenden raspelartigen Werkzeuge einen ovalen oder länglichen Querschnitt, wobei die lange Achse in Richtung des Schenkelhalses der Femurprothese weist, also in Richtung auf die Implantat-Gelenkkugel. Wenn diese Längsmittelebene des Schaftes als Instrumentenebene verwendet wird, ist sichergestellt, dass diese Ebene durch die Implantat-Gelenkkugel geht und damit dem Operateur anzeigt, wie die Richtung vom Schaft zum Implantat-Gelenkpunkt in der Transversalebene verläuft.

Um dem Operateur die Funktion des Mittelpunktes der Gelenkkugel des natürlichen Femurkopfes deutlich zu machen, kann es gemäß einer bevorzugten Ausführungsform vorgesehen sein, dass man in der Darstellung der Transversalebene des Femurs und der Schnittlinie ein Bild eines Transversalschnittes der Pelvis in Höhe des Mittelpunktes des die Implantat-Gelenkkugel aufnehmenden Acetabulums so einkopiert, dass der Mittelpunkt der Gelenkkugel des natürlichen Femurkopfes und der Mittelpunkt des Acetabulums zusammenfallen. Der Transversalschnitt ist dabei eine Ebene, die durch die beiden Mittelpunkte der Acetabulen hindurchgeht und bei aufrecht stehendem Patienten horizontal verläuft. Es kann sich dabei um eine Querschnittsansicht handeln, aber auch um eine Draufsicht auf die Pelvis, bei der die Lage des Mittelpunktes des Acetabulums dadurch sichtbar gemacht wird, dass die Pelvis teilweise durchsichtig dargestellt wird. Wesentlich ist nur, dass dem Operateur durch diese Darstellung symbolisch gezeigt wird, dass der Mittelpunkt der Gelenkkugel des natürlichen Femurkopfes im Acetabulum der Pelvis angeordnet ist.

Der Operateur kann in der beschriebenen Darstellung bei der Änderung der Position und Orientierung des Werkzeuges und/oder des Femurimplantates sofort sehen, wie sich die Schnittlinie zwischen Instrumentenebene und Transversalebene des Femurs verschiebt, bei einer Drehung des Schaftes des raspelähnlichen Werkzeuges oder des Femurimplantates wird sich der Winkel dieser Schnittlinie in der Darstellung verändern, bei einer seitlichen Verschiebung des Werkzeugschaftes oder des Implantatschaftes ohne Drehung wird sich die Linie parallel zu sich selbst verschieben. Eine korrekte Position und Orientierung des Werkzeugschaftes und des Femurimplantatschaftes erhält man dann, wenn die Schnittlinie durch das Bild des Mittelpunkts der Gelenkkugel des natürlichen Femurkopfes hindurchgeht. Da die Instrumentenebene selbst durch den Mittelpunkt der Implantat-Gelenkkugel hindurchgeht, zeigt dies dem Operateur an, dass sowohl die Winkelorientierung als auch die seitliche Verschiebung des Werkzeugschaftes oder des Femurimplantatschaftes korrekt gewählt sind.

Die nachfolgende Beschreibung bevorzugter Ausführungsformen der Erfindung dient im Zusammenhang mit der Zeichnung der näheren Erläuterung. Es zeigen:

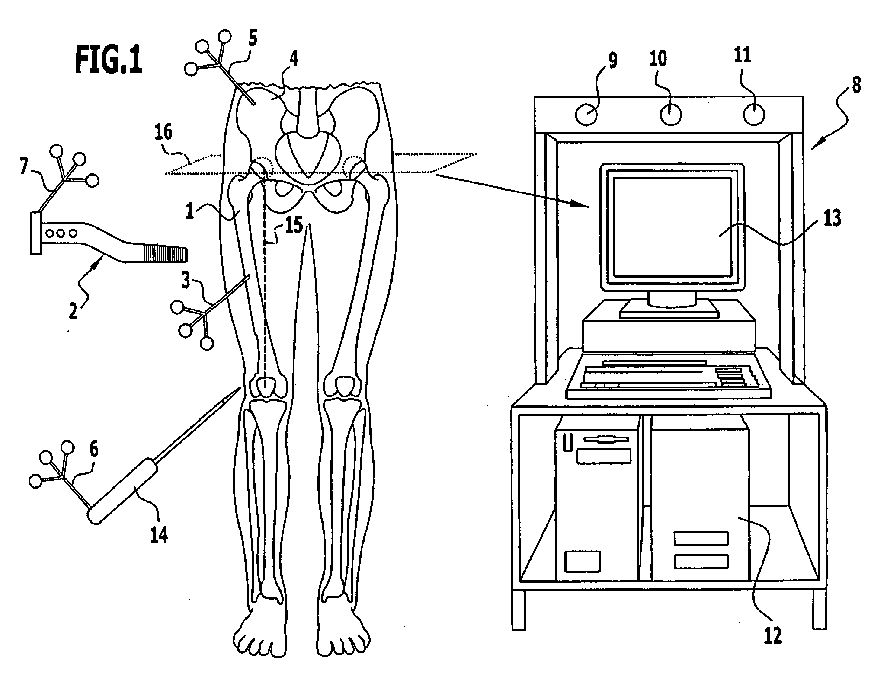

- Figur 1:: eine schematische Ansicht der Pelvis und des Femurs eines Pati- enten sowie ein Navigationssystem mit einer Datenverarbeitungs- anlage und mit einer Anzeige;

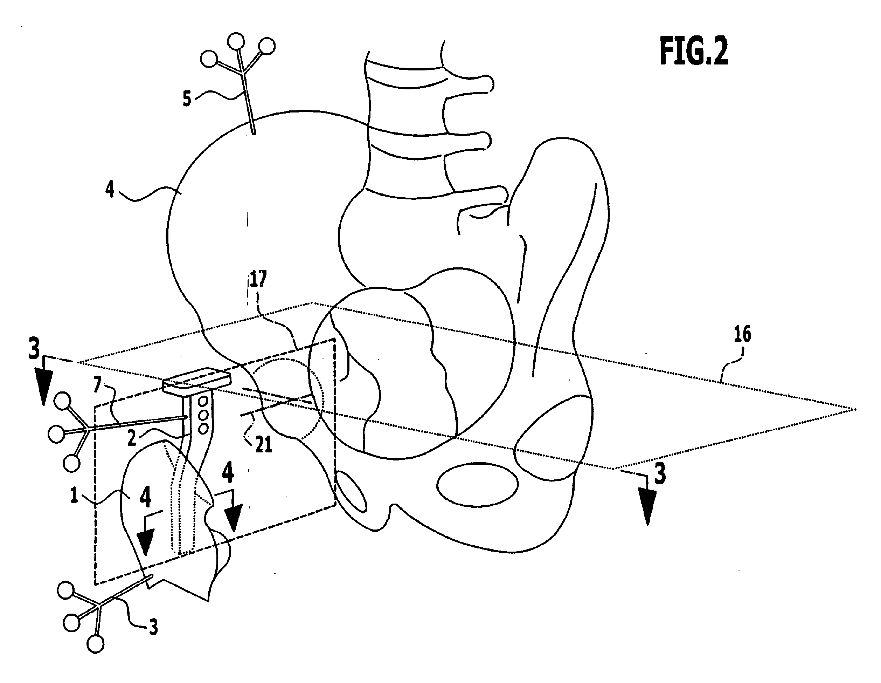

- Figur 2:: eine schematische Ansicht der Pelvis und eines Teils des Femurs mit einem eingeführten und navigierten raspelähnlichen Werk- zeug unter Angabe der femuralen Transversalebene und der In- strumentenebene;
- Figur 3:: eine Ansicht der Anzeige mit einer Darstellung der Schnittlinie der femuralen Transversalebene und der Instrumentenebene, dem Mittelpunkt der Gelenkkugel des natürlichen Femurkopfes und ei- ner einkopierten Darstellung der Pelvis;
- Figur 4:: eine Querschnittsdarstellung des Schaftes eines raspelähnlichen Werkzeuges gemäß Linie 4-4 in Figur 2;
- Figur 5:: eine schematische Seitenansicht eines Femurimplantates mit ei- nem Schaft, einem Schenkelhals und einer Implantat-Gelenkkugel und
- Figur 6:: eine Schnittansicht des Femurimplantates der Figur 5 längs Li- nie 6-6 in Figur 5.

Die erfindungsgemäße Vorrichtung wird verwendet, um den Femur 1 eines Patienten für die Aufnahme eines Femurimplantates vorzubereiten oder ein Femurimplantat in diesen einzusetzen. Dies ist notwendig bei einer Hüftgelenksoperation, bei der das natürliche Hüftgelenk ersetzt wird durch eine künstliche Gelenkpfanne in der Pelvis und ein Femurimplantat mit einer Implantat-Gelenkkugel, die nach Entfernung des natürlichen Femurkopfes an dessen Stelle in den verbleibenden Femur eingesetzt wird. Die Femurimplantate weisen normalerweise einen Schaft auf, der in den Markraum des Femurs eingetrieben wird, und dieser Markraum muss dazu entsprechend vorbereitet werden. Diese Vorbereitung erfolgt mit Hilfe eines raspelähnlichen Werkzeuges 2, welches dem Schaft des Femurimplantates sehr ähnlich ausgebildet ist und den Markraum des Femurs 1 formgerecht so vorbereitet, dass beim Einführen des Schaftes des Femurimplantates die Positionierung des Femurimplantates im Femur durch die Geometrie des vorbereiteten Markraumhohlraumes bestimmt wird. Es ist daher sehr wichtig, dass das Werkzeug 2 bei der Vorbereitung des Femurs in genau der gewünschten Winkelorientierung und seitlichen Lage in den Femur eingetrieben wird, außerdem ist es natürlich wesentlich, das Femurimplantat in der richtigen Lage in den Femur einzusetzen. Richtige Lage bedeutet dabei, dass der seitlich abstehende Arm der Femurkomponente, der üblicherweise an seinem Ende eine Implantat-Gelenkkugel trägt, relativ zur Pelvis und zur Gelenkpfanne in der Pelvis die richtige Orientierung einnimmt.

Aufgrund der Vorbereitung der Operation weiß der Operateur, wo relativ zum natürlichen Femur der Gelenkkopf des Femurimplantates, also die Implantat-Gelenkkugel, zu platzieren ist, außerdem sind die geometrischen Daten des Femurimplantates bekannt, so dass beim Einsetzen des Femurimplantates in den Femur in jedem Falle feststellbar ist, welche Position des Femurimplantates im Femur zu welcher Position des Implantat-Gelenkkopfes führt.

Um dies überprüfen zu können, werden am Femur 1 ein Markierelement 3, an der Pelvis 4 ein Markierelement 5 und an dem Werkzeug 2 ein Markierelement 7 dauerhaft und starr befestigt, wobei das Werkzeug 2 in gleicher Weise ersetzt werden könnte durch das Femurimplantat. Im folgenden wird daher ausschließlich vom Werkzeug 2 gesprochen werden.

Die Lage der Markierelemente 3, 5 und 7 kann mittels eines an sich bekannten Navigationssystems 8 bestimmt werden, das beispielsweise drei räumlich voneinander getrennte Sender 9, 10, 11 aufweist, die eine Infrarotstrahlung aussenden, die von räumlich getrennten Reflektoren der Markierelemente 3, 5, 7 reflektiert und von den auch als Empfänger arbeitenden Sendern 9, 10, 11 wieder aufgefangen wird. Aus der Strahlung können sowohl die Orientierung als auch die Position der Markierelemente im Raum errechnet werden, also die Lage jedes einzelnen Markierelementes. Da die Markierelemente starr mit dem Femur, der Pelvis bzw. dem Werkzeug verbunden sind, ist damit auch die jeweilige Lage der Markierelemente im Raum jederzeit zu bestimmen.

Die entsprechenden Lagedatensätze werden von dem Navigationssystem 8 einer Datenverarbeitungsanlage 12 zugeführt, die diese Daten zur Berechnung weiterer Daten verwendet und unter anderem auf einem Bildschirm 13 eine optische Anzeige erzeugen kann.

Mit Hilfe der beschriebenen Vorrichtung wird zunächst die Lage des Mittelpunktes der Gelenkkugel des natürlichen Femurkopfes relativ zum Femur bestimmt. Diese Bestimmung erfolgt vor der Entfernung des natürlichen Femurkopfes einfach dadurch, dass der Femur relativ zur Pelvis verschwenkt wird. Bei dieser Verschwenkbewegung bewegt sich das Markierelement 3 des Femurs 1 auf einer Kugelschale, deren Mittelpunkt der Mittelpunkt der Gelenkkugel des natürlichen Femurkopfes ist. Die Datenverarbeitungsanlage kann aus dieser Bewegung und den entsprechenden Lagedatensätzen den Mittelpunkt der Gelenkkugel des natürlichen Femurkopfes relativ zum Markierelement 3 und damit relativ zum Femur 1 berechnen.

Außerdem wird die Lage des Kniegelenks bestimmt, dies kann durch Palpation mit Hilfe eines mit einem Markierelement 6 versehenen Tastelementes 14 erfolgen, das mit seiner Spitze beispielsweise an die Kniescheibe des Patienten angelegt wird. Die Datenverarbeitungsanlage kann aus den Lagedaten des navigierten Tastelements 14 und den geometrischen Daten desselben die genaue Lage des palpierten Markierpunktes an der Kniescheibe berechnen, der als Kniegelenkpunkt verwendet wird. Die Datenverarbeitungsanlage bildet dann die Verbindungslinie des Mittelpunkts der Gelenkkugel des natürlichen Femurkopfes, also der Femur-Gelenkkugel, und des Kniegelenkes, diese Verbindungslinie wird als mechanische Femurachse 15 bezeichnet.

Aus dieser mechanischen Femurachse 15 und der Lage des Mittelpunkts der Gelenkkugel des natürlichen Femurkopfes berechnet die Datenverarbeitungsanlage 12 weiterhin eine senkrecht auf der mechanischen Femurachse 15 stehende und durch den Mittelpunkt der Gelenkkugel des natürlichen Femurkopfes hindurchgehende femurale Transversalebene 16, es handelt sich dabei um eine dem Femur zugeordnete Schnittebene.

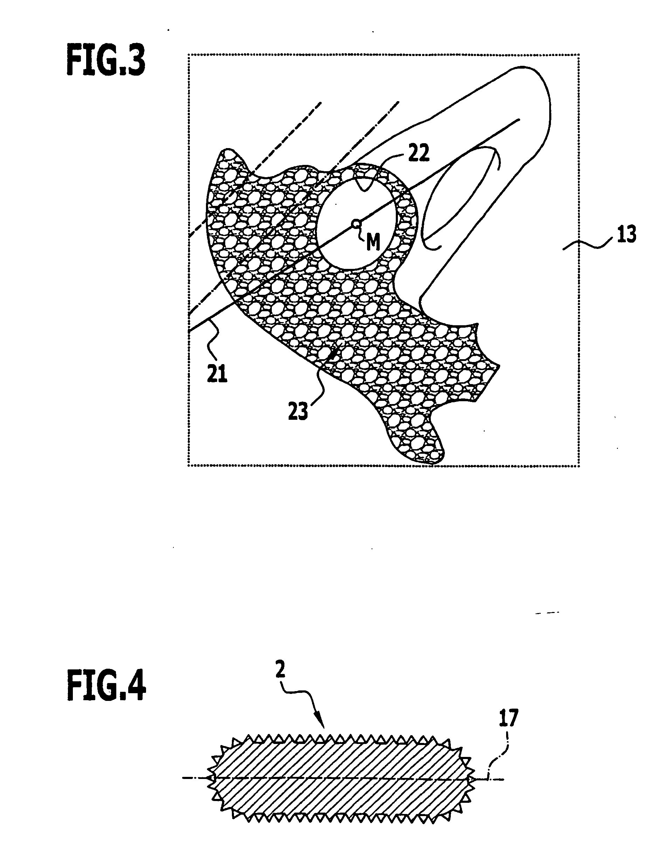

Dem Werkzeug 2 wird eine Instrumentenebene 17 zugeordnet. Dabei kann es sich beispielsweise um eine Längsmittelebene handeln. Wie aus der Darstellung der Figur 4 deutlich wird, ist der Schaft beispielsweise oval ausgebildet mit einer langen und einer kurzen Achse, die Längsmittelebene verläuft längs der langen Achse und in Längsrichtung des Schaftes.

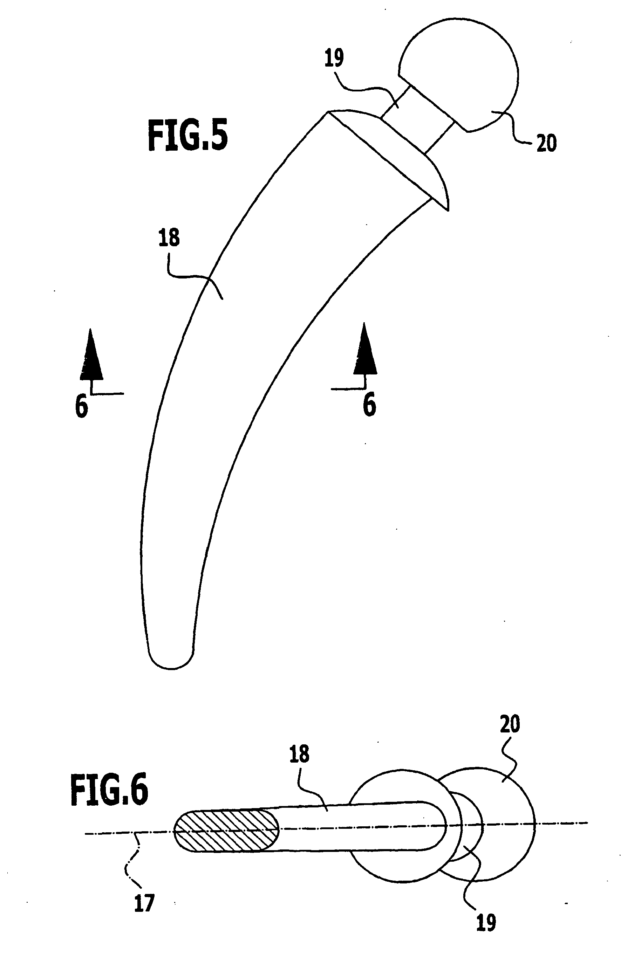

Wenn ein Femurimplantat mit ähnlicher Schaftform verwendet wird, kann vorgesehen sein, dass der sich an den Schaft 18 anschließenden Schenkelhals 19, der die Implantat-Gelenkkugel 20 trägt, ebenfalls in der Längsmittelebene des Femurimplantats verläuft, wie dies in Figur 6 dargestellt ist. Es kann aber auch vorgesehen sein, dass dieser Verbindungsarm einen Winkel gegenüber dieser Ebene einschließt. Beim formgerechten Einsetzen des Schaftes in eine von dem Werkzeug vorbereitete Öffnung liegt dann der Schenkelhals nicht in der Längsmittelebene des Instrumentes, sondern in der durch den Verbindungsarm zwischen Schaft und Implantat-Gelenkkugel 20 aufgespannten Ebene, und diese bildet die von der Datenverarbeitungsanlage verwendete Instrumentenebene.

Die Datenverarbeitungsanlage erhält vom Navigationssystem jeweils die Lagedaten des Werkzeuges 2 und berechnet daraus die Lage der jeweiligen Instrumentenebene und weiterhin die Schnittlinie 21 der Instrumentenebene 17 und der femuralen Transversalebene 16. Auf der Anzeige wird diese Schnittlinie in der femuralen Transversalebene 16 angezeigt, außerdem der Mittelpunkt M der Gelenkkugel des natürlichen Femurkopfes.

Wenn diese Schnittlinie 21 durch den Mittelpunkt M der Gelenkkugel des natürlichen Femurkopfes hindurchgeht, sind das Werkzeug 2 oder das Femurimplantat richtig orientiert, es ist nämlich dann sichergestellt, dass die Instrumentenebene durch den Mittelpunkt M der Gelenkkugel des natürlichen Femurkopfes hindurchgeht. Wenn dagegen Abweichungen vorliegen, weiß der Operateur, dass er das Werkzeug oder das Femurimplantat seitlich verschieben oder drehen muss, um die gewünschte Lage zu erreichen.

Zusätzlich wird auf dem Bildschirm 13 eine Draufsicht oder eine horizontale Querschnittansicht einer Pelvis abgebildet, diese Pelvisdarstellung 23 kann schematisch sein und benötigt nicht die anatomischen Daten des Patienten, sie dient lediglich dazu, dem Operateur anzuzeigen, dass es sich bei dem Mittelpunkt M gleichzeitig auch um den Mittelpunkt der die Gelenkkugel aufnehmenden Gelenkpfanne handelt. Zu diesem Zweck ist diese Gelenkpfanne 22 in der Schnittdarstellung oder in einer Transparentdarstellung sichtbar. Die Pelvisdarstellung 23 wird dabei so angeordnet, dass der Mittelpunkt der Gelenkpfanne mit dem Mittelpunkt M der Gelenkkugel des natürlichen Femurkopfes zusammenfällt.

Normalerweise wird die femurale Transversalebene nicht mit der Schnittebene zusammenfallen, die sich bei einer horizontalen durch die Hüftgelenkmittelpunkte verlaufenden Querschnittsansicht der Pelvis ergibt.

Es besteht aber die Möglichkeit, aus der Lage der femuralen Transversalebene relativ zu dieser horizontalen Querschnittsebene einen Datensatz zu bestimmen, der beschreibt, wie der Femur und die mit dem Femur verbundenen Teile verschoben und verschwenkt werden müssen, damit die femurale Transversalebene mit dieser horizontalen Querschnittsebene, die als Transversalebene der Pelvis bezeichnet wird, zusammenfällt. Wenn der Femur in einer Stellung steht, bei der diese beiden Ebenen zusammenfallen, spricht man von einer neutralen Position des Femurs.

Unter Verwendung dieses Transformationsdatensatzes wäre es dann auch möglich, statt eines Schnittes in der Transversalebene des Femurs einen Schnitt in der Transversalebene der Pelvis darzustellen, da beide Ebenen in der neutralen Position zusammenfallen. Voraussetzung ist aber, dass die die Position des Femurs und der damit starr verbundenen Teile angebenden Positions- und Orientierungsdaten so umgerechnet werden, dass sich der Femur relativ zur Pelvis in der neutralen Position befindet.

## Patentansprüche

1. Vorrichtung zur Anzeige der Position und Orientierung eines raspelähnlichen Werkzeuges (2) zur Vorbereitung eines Femurs (1) für ein mit einer Implantat-Gelenkkugel (20) in das Acetabulum der Pelvis (4) oder in eine in die Pelvis (4) eingesetzte Gelenkpfanne eingreifendes Femurimplantat,
umfassend
ein derartiges Werkzeug (2), wobei das raspelähnliche Werkzeug dem Schaft des Femurimplantates sehr ähnlich ausgebildet und geeignet ist, die Markhöhle formgerecht so vorzubereiten, dass beim Einführen des Schaftes des Femurimplantates die Positionierung des Femurimplantates im Femur durch die Geometrie des vorbereiteten Markraumhohlraumes bestimmt wird,
ein Navigationssystem (8) mit dem Femur (1), der Pelvis (4) sowie dem Werkzeug (2) zugeordneten Markierelementen (3, 5, 7) zur Lagebestimmung dieser Teile,
und eine dem Navigationssystem (8) zugeordnete Datenverarbeitungsanlage (12), die geeignet ist, eine Anzeige zu aktivieren, **dadurch gekennzeichnet,**
**dass** die Datenverarbeitungsanlage (12) so programmiert ist, dass sie zum Auffinden einer Transversalebene des Femurs (16) aus der Position und Orientierung des Mittelpunktes (M) der Gelenkkugel des natürlichen Femurkopfes und des Kniegelenks als Verbindungslinie dieser beiden Punkte eine mechanische Femurachse (15) bestimmt und die Transversalebene des Femurs (16) als senkrecht auf der mechanischen Femurachse (15) und durch den Mittelpunkt (M) der Gelenkkugel des natürlichen Femurkopfes gehend bestimmt,
und **dass** sie auf der Anzeige (13) in dieser Transversalebene des Femurs (16) in Höhe des Mittelpunktes (M) der Gelenkkugel des natürlichen Femurkopfes diesen Mittelpunkt (M) und eine Schnittlinie (21) der Transversalebene des Femurs (16) und einer Instrumentenebene (17) darstellt,
wobei die Datenverarbeitungsanlage (12) so programmiert ist, dass sie die Instrumentenebene (17) so auswählt, dass die Projektion der Längsachse eines den Schaft des raspelähnlichen Werkzeuges (2) mit der Implantat-Gelenkkugel (20) verbindenden Haltearmes (19) auf die Transversalebene des Femurs (16) in der Instrumentenebene (17) liegt und dass die Instrumentenebene (17) durch den Mittelpunkt der Implantat-Gelenkkugel (20) hindurchgeht.

2. Vorrichtung zur Anzeige der Position und Orientierung eines in einen Femur (1) einzusetzenden Femurimplantates relativ zum Femur (1) und zum Mittelpunkt (M) der Femur-Gelenkkugel, umfassend ein derartiges Femurimplantat,
ein Navigationssystem (8) mit dem Femur (1), der Pelvis (4) und dem Femurimplantat zugeordneten Markierelementen (3, 5) zur Lagebestimmung dieser Teile,
und mit einer dem Navigationssystem (8) zugeordneten Datenverarbeitungsanlage (12), die eine Anzeige aktiviert, **dadurch gekennzeichnet, dass** die Datenverarbeitungsanlage (12) so programmiert ist, dass sie zum Auffinden einer Transversalebene des Femurs (16) aus der Position und Orientierung des Mittelpunktes (M) der Gelenkkugel des natürlichen Femurkopfes und des Kniegelenks als Verbindungslinie dieser beiden Punkte eine mechanische Femurachse (15) bestimmt und die Transversalebene des Femurs (16) als senkrecht auf der mechanischen Femurachse (15) und durch den Mittelpunkt (M) der Gelenkkugel des natürlichen Femurkopfes gehend bestimmt,
und dass sie auf der Anzeige (13) in dieser Transversalebene des Femurs (16) in Höhe des Mittelpunktes (M) der Gelenkkugel des natürlichen Femurkopfes diesen Mittelpunkt (M) und eine Schnittlinie (21) der Transversalebene des Femurs (16) und einer Instrumentenebene (17) darstellt,
wobei die Datenverarbeitungsanlage (12) so programmiert ist, dass sie die Instrumentenebene (17) so auswählt, dass die Projektion der Längsachse eines den Schaft (18) des Femurimplantates mit der Implantat-Gelenkkugel (20) verbindenden Haltearmes (19) auf die Transversalebene des Femurs (16) in der Instrumentenebene (17) liegt und dass die Instrumentenebene (17) durch den Mittelpunkt der Implantat-Gelenkkugel (20) hindurchgeht.

3. Vorrichtung nach Anspruch 1, **dadurch gekennzeichnet, dass** die Datenverarbeitungsanlage (12) so programmiert ist, dass sie die Mittelebene des raspelähnlichen Werkzeuges (2) als Instrumentenebene (17) wählt.

4. Vorrichtung nach einem der Ansprüche 1 bis 3, **dadurch gekennzeichnet, dass** die Datenverarbeitungsanlage (12) so programmiert ist, dass sie in der Darstellung der Transversalebene des Femurs (16) und der Schnittlinie (21) ein Bild (23) eines Transversalschnittes der Pelvis in Höhe des Mittelpunktes des die Implantat-Gelenkkugel (20) aufnehmenden Acetabulums so einkopiert, dass der Mittelpunkt (M) der Gelenkkugel des natürlichen Femurkopfes und der Mittelpunkt des Acetabulums zusammenfallen.

## Claims

1. Apparatus for indicating the position and orientation of a rasp-like tool (2) for preparation of a femur (1) for a femur implant which with an implant joint ball (20) fits into the acetabulum of the pelvis (4) or into a joint socket inserted in the pelvis (4), comprising such a tool (2), the rasp-like tool being constructed very similarly to the shaft of the femur implant and being suited to prepare the medullary cavity appropriately so that on introducing the shaft of the femur implant, the positioning of the femur implant in the femur is determined by the geometry of the prepared medullary cavity,
a navigation system (8) with marking elements (3, 5, 7) associated with the femur (1), the pelvis (4) and the tool (2) for determining the position of these parts, and
a data processing installation (12) associated with the navigation system (8), which is suited for activating a display, **characterized in that**
in order to locate a transversal plane of the femur (16), the data processing installation (12) is programmed to determine a mechanical femur axis (15) from the position and orientation of the center point (M) of the joint ball of the natural femur head and of the knee joint as line connecting these two points, and to determine the transversal plane of the femur (16) as extending perpendicularly to the mechanical femur axis (15) and passing through the center point (M) of the joint ball of the natural femur head, and to represent on the display (13), in this transversal plane of the femur (16) at the level of the center point (M) of the joint ball of the natural femur head, this center point (M) and a line of intersection (21) of the transversal plane of the femur (16) and an instrument plane (17),
the data processing installation (12) being programmed to select the instrument plane (17) such that the projection of the longitudinal axis of a holding arm (19) connecting the shaft of the rasp-like tool (2) to the implant joint ball (20) onto the transversal plane of the femur (16) lies in the instrument plane (17), and such that the instrument plane (17) passes through the center point of the implant joint ball (20).

2. Apparatus for indicating the position and orientation of a femur implant to be inserted into a femur (1) relative to the femur (1) and to the center point (M) of the femur joint ball, comprising such a femur implant,
a navigation system (8) with marking elements (3, 5) associated with the femur (1), the pelvis (4) and the femur implant for determining the position of these parts, and
a data processing installation (12) associated with the navigation system (8), which activates a display, **characterized in that** in order to locate a transversal plane of the femur (16), the data processing installation (12) is programmed to determine a mechanical femur axis (15) from the position and orientation of the center point (M) of the joint ball of the natural femur head and of the knee joint as line connecting these two points, and to determine the transversal plane of the femur (16) as extending perpendicularly to the mechanical femur axis (15) and passing through the center point (M) of the joint ball of the natural femur head, and to represent on the display (13), in this transversal plane of the femur (16) at the level of the center point (M) of the joint ball of the natural femur head, this center point (M) and a line of intersection (21) of the transversal plane of the femur (16) and an instrument plane (17),
the data processing installation (12) being programmed to select the instrument plane (17) such that the projection of the longitudinal axis of a holding arm (19) connecting the shaft (18) of the femur implant to the implant joint ball (20) onto the transversal plane of the femur (16) lies in the instrument plane (17), and such that the instrument plane (17) passes through the center point of the implant joint ball (20).

3. Apparatus in accordance with claim 1, **characterized in that** the data processing installation (12) is programmed to select the center plane of the rasp-like tool (2) as instrument plane (17).

4. Apparatus in accordance with any one of claims 1 to 3, **characterized in that** the data processing installation (12) is programmed to copy into the representation of the transversal plane of the femur (16) and the line of intersection (21) an image (23) of a transversal section of the pelvis at the level of the center point of the acetabulum receiving the implant joint ball (20) such that the center point (M) of the joint ball of the natural femur head and the center point of the acetabulum coincide.

## Revendications

1. Dispositif destiné à visualiser la position et l'orientation d'un outil (2) du type râpe, en vue de la préparation d'un fémur (1) pour un implant de fémur s'engageant avec une sphère d'articulation d'implant (20) dans l'acetabulum du bassin ou de l'os iliaque (4), ou dans une cupule d'articulation implantée dans l'os iliaque (4),
comprenant
un tel outil (2), l'outil du type râpe étant d'une configuration très similaire à la tige de l'implant de fémur et étant adapté à préparer la cavité médullaire de façon telle, que lors de l'introduction de la tige de l'implant de fémur, le positionnement de l'implant de fémur dans le fémur soit déterminé par la géométrie de la cavité médullaire ayant été préparée,
un système de navigation (8) avec des éléments de marquage (3, 5, 7) associés au fémur (1), à l'os iliaque (4) ainsi qu'à l'outil (2), pour la détermination du positionnement de ces parties,
et un ensemble de traitement de données (12) associé au système de navigation (8) et adapté à activer un système de visualisation,
**caractérisé en ce que** l'ensemble de traitement de données (12) est programmé
de manière à déterminer, en vue de trouver un plan transversal de fémur (16), à partir de la position et de l'orientation du point central (M) de la sphère d'articulation de la tête de fémur naturelle et de l'articulation du genou, en tant que ligne de liaison des ces deux points, un axe mécanique de fémur (15), et à définir ensuite le plan transversal de fémur (16) comme étant un plan perpendiculaire à l'axe mécanique de fémur (15) et passant par le point central (M) de la sphère d'articulation de la tête de fémur naturelle,
et de manière à représenter sur le système de visualisation (13), dans ce plan transversal de fémur (16), au niveau du point central (M) de la sphère d'articulation de la tête de fémur naturelle, ce point central (M) et une ligne d'intersection (21) du plan transversal de fémur (16) et d'un plan d'instrument (17),
l'ensemble de traitement de données (12) étant programmé de manière à sélectionner le plan d'instrument (17) de façon à ce que la projection sur le plan transversal de fémur (16), de l'axe longitudinal d'un bras de support (19), qui relie la tige ou le corps de l'outil (2) du type râpe à la sphère d'articulation d'implant (20), soit située dans le plan d'instrument (17), et que le plan d'instrument (17) passe par le point central de la sphère d'articulation d'implant (20).

2. Dispositif destiné à visualiser la position et l'orientation d'un implant de fémur à mettre en place dans un fémur (1), par rapport au fémur (1) et au point central (M) de la sphère d'articulation de fémur, comprenant
un tel implant de fémur,
un système de navigation (8) avec des éléments de marquage (3, 5) associés au fémur (1), à l'os iliaque (4) et à l'implant de fémur, pour la détermination du positionnement de ces parties,
et un ensemble de traitement de données (12), qui est associé au système de navigation (8) et active un système de visualisation,
**caractérisé en ce que** l'ensemble de traitement de données (12) est programmé
de manière à déterminer, en vue de trouver un plan transversal de fémur (16), à partir de la position et de l'orientation du point central (M) de la sphère d'articulation de la tête de fémur naturelle et de l'articulation du genou, en tant que ligne de liaison des ces deux points, un axe mécanique de fémur (15), et à définir ensuite le plan transversal de fémur (16) comme étant un plan perpendiculaire à l'axe mécanique de fémur (15) et passant par le point central (M) de la sphère d'articulation de la tête de fémur naturelle,
et de manière à représenter sur le système de visualisation (13), dans ce plan transversal de fémur (16), au niveau du point central (M) de la sphère d'articulation de la tête de fémur naturelle, ce point central (M) et une ligne d'intersection (21) du plan transversal de fémur (16) et d'un plan d'instrument (17),
l'ensemble de traitement de données (12) étant programmé de manière à sélectionner le plan d'instrument (17) de façon à ce que la projection sur le plan transversal de fémur (16), de l'axe longitudinal d'un bras de support (19), qui relie la tige (18) de l'implant de fémur à la sphère d'articulation d'implant (20), soit située dans le plan d'instrument (17), et que le plan d'instrument (17) passe par le point central de la sphère d'articulation d'implant (20).

3. Dispositif selon la revendication 1, **caractérisé en ce que** l'ensemble de traitement de données (12) est programmé de manière à sélectionner le plan médian de l'outil (2) du type râpe, en tant que plan d'instrument (17).

4. Dispositif selon l'une des revendications 1 à 3, **caractérisé en ce que** l'ensemble de traitement de données (12) est programmé de manière à copier dans la représentation du plan transversal de fémur (16) et de la ligne d'intersection (21), une image (23) d'une coupe transversale de l'os iliaque à hauteur du point central de l'acetabulum recevant la sphère d'articulation d'implant (20), de façon à ce que le point central (M) de la sphère d'articulation de la tête de fémur naturelle et le point central de l'acetabulum soient confondus.
